(19) Europäisches Patentamt
European Patent Office
Office européen des brevets

(11) Veröffentlichungsnummer: **0 011 191**
**A1**

# EUROPÄISCHE PATENTANMELDUNG

(21) Anmeldenummer: **79104254.2**

(22) Anmeldetag: **02.11.79**

(51) Int. Cl.³: **C 07 D 233/60**
A 61 K 31/415

(30) Priorität: **18.11.78 DE 2850057**

(43) Veröffentlichungstag der Anmeldung:
**28.05.80 Patentblatt 80 11**

(84) Benannte Vertragsstaaten:
**AT BE CH DE FR GB IT LU NL SE**

(71) Anmelder: **BAYER AG**
**Zentralbereich Patente, Marken und Lizenzen**
**D-5090 Leverkusen 1, Bayerwerk(DE)**

(72) Erfinder: **Krämer, Wolfgang, Dr.**
**Am Eckbusch 39/162**
**D-5600 Wuppertal-1(DE)**

(72) Erfinder: **Büchel, Karl Heinz, Dr.**
**Bergerheide 62**
**D-5600 Wuppertal-1(DE)**

(72) Erfinder: **Haller, Ingo, Dr.**
**Viktoriastrasse 99**
**D-5600 Wuppertal-1(DE)**

(72) Erfinder: **Plempel, Manfred, Dr.**
**Pahlkestrasse 5**
**D-5600 Wuppertal-1(DE)**

(54) **Diastereomeres 1-(4-Chlorphenoxy)-1-(1-imidazolyl)-3,3-dimethyl-2-butanol, Verfahren zu seiner Herstellung sowie seine Verwendung als Arzneimittel.**

(57) Das Enantiomerenpaar der diastereomeren Form A des 1-(4 -Chlorphenoxy)-1-(1 -imidazolyl)-3,3 -dimethyl-2-butanol der Formel

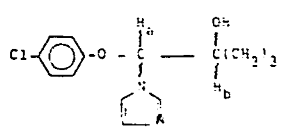

(I)

und deren physiologisch verträglichen Säureadditions-Salze weisen starke antimykotische Eigenschaften auf, die diejenige des bekannten Diastereomerengemisches bei weitem übertrifft.

EP 0 011 191 A1

0011191

BAYER AKTIENGESELLSCHAFT 5090 Leverkusen, Bayerwerk
Zentralbereich
Patente, Marken und Lizenzen Si/Kü
Ia

# Diastereomeres 1-(4-Chlorphenoxy)-1-(1-imidazolyl)-3,3-dimethyl-2-butanol, Verfahren zu seiner Herstellung sowie seine Verwendung als Arzneimittel

Die vorliegende Erfindung betrifft das neue diastereomere 1-(4-Chlorphenoxy)-1-(1-imidazolyl)-3,3-dimethyl-2-butanol, ein Verfahren zu seiner Herstellung sowie seine Verwendung als Arzneimittel, insbesondere als Antimykotikum.

Es ist bereits bekannt geworden, daß das Diastereomerengemisch von 1-(4-Chlorphenoxy)-1-(1-imidazolyl)-3,3-dimethyl-2-butanol allgemein antimykotische Wirkung aufweist (vergleiche DE-OS 23 33 355 bzw. US-Patentschrift 3 968 229 [LeA 15 145]).

Es wurde nun als neue Verbindung das Enantiomerenpaar der diastereomeren Form A (siehe nachfolgende Erläuterung) des 1-(4-Chlorphenoxy)-1-(1-imidazolyl)-3,3-dimethyl-2-butanol der Formel

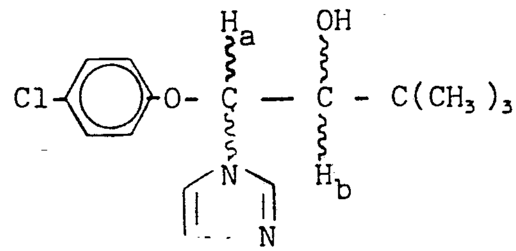

(I)

und deren physiologisch verträglichen Säureadditions-Salze gefunden. Sie weisen starke antimykotische Eigenschaften auf.

Zur Erläuterung sei hierzu vermerkt:
Verbindungen mit zwei asymmetrischen Kohlenstoffatomen können in den beiden diastereomeren Formen threo und erythro vorliegen. Bei der erfindungsgemäßen Verbindung ist die Zuordnung aufgrund der noch nicht bestimmten absoluten Konfiguration nur bedingt möglich, dies wird durch die Wellenlinien in Formel (I) zum Ausdruck gebracht. Entsprechend wird hier in Form A und Form B unterteilt, die sich nach ihren physikalisch-chemischen Eigenschaften eindeutig charakterisieren lassen, wobei die hydrophilere Form, charakterisiert durch kleinere Kopplungskonstanten der Protonen $H_{(a)}$ und $H_{(b)}$ im NMR-Spektrum, als Form A bezeichnet wird.

Weiterhin wurde gefunden, daß man die diastereomere Form A des 1-(4-Chlorphenoxy)-1-(1-imidazolyl)-3,3-dimethyl-2-butanol der Formel (I) erhält, wenn man 1-(4-Chlorphenoxy)-1-(1-imidazolyl)-3,3-dimethyl-2-butanon der Formel

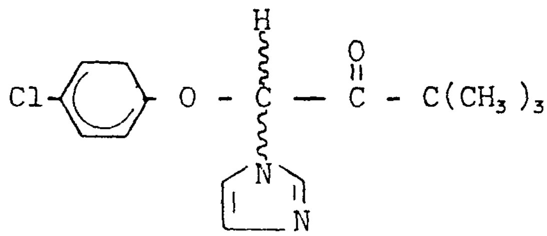

(II)

mit sekundären Alkoholaten in Gegenwart eines Verdünnungsmittels stereoselektiv reduziert.

Weiterhin kann die erfindungsgemäß erhältliche Form A des 1-(4-Chlorphenoxy)-1-(1-imidazolyl)-3,3-dimethyl-2-butanols der Formel (I) durch Umsetzen mit Säuren in die Salze überführt werden.

Ueberraschenderweise zeigt die erfindungsgemäße diastereomere Form A des 1-(4-Chlorphenoxy)-1-(1-imidazolyl)-3,3-dimethyl-2-butanols der Formel (I) eine erheblich bessere, therapeutisch nutzbare Wirksamkeit als das aus dem Stand der Technik bekannte entsprechende Diastereomerengemisch. Die erfindungsgemäßen Stoffe stellen somit eine wertvolle Bereicherung der Pharmazie dar.

Aufgrund des bekannten Standes der Technik konnte keineswegs erwartet werden, daß sich die erfindungsgemäße Form A der Formel (I) durch sehr gute antimykotische Eigenschaften auszeichnet, während die analoge Form B dieser Verbindung als Antimykotikum nur schwach aktiv ist.

Verwendet man neben dem 1-(4-Chlorphenoxy)-1-(1-imidazolyl)-3,3-dimethyl-2-butanon Aluminiumisopropylat als Ausgangsstoff , so kann der Reaktionsablauf durch das folgende Formelschema wiedergegeben werden:

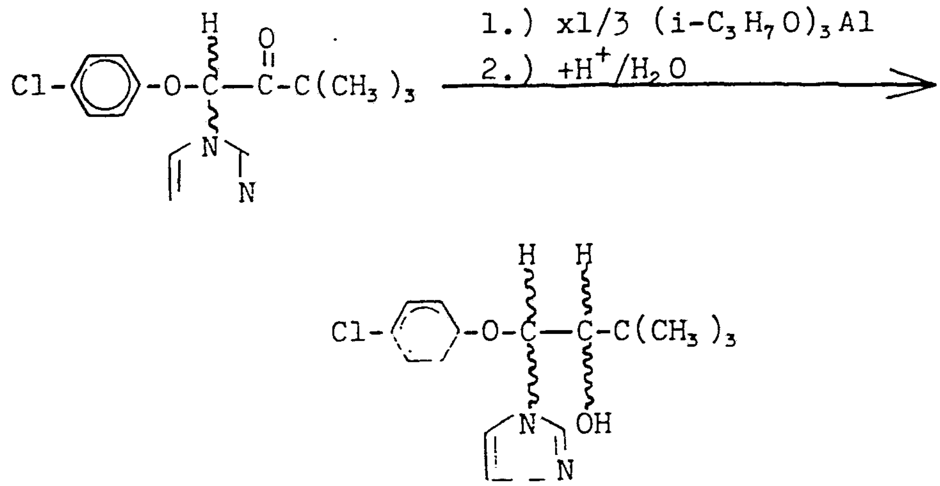


Das als Ausgangsstoff zu verwendende Keton der Formel (II) ist bekannt (vergleiche DE-OS 21 05 490 [LeA 13 458]). Man erhält es nach dem dort beschriebenen Verfahren, indem man 1-(4-Chlorphenoxy)-1-halogen-3,3-dimethyl-2-butanon mit Imidazol, gegebenenfalls in Gegenwart eines Säurebindemittels, bei Temperaturen zwischen 60 und 120°C umsetzt.

Die erfindungsgemäße Reduktion wird mit Hilfe von sekundären Alkoholaten durchgeführt. Hierzu gehören vorzugsweise die sekundären Alkoholate des Aluminiums, wie insbesondere Aluminiumisopropylat, Aluminium-sek.-butylat und Aluminiumcyclohexylat.

Für die erfindungsgemäße Umsetzung kommen als Verdünnungsmittel vorzugsweise inerte organische Lösungsmittel infrage. Hierzu gehören vorzugsweise Alkohole, wie insbesondere Isopropanol und sek. Butanol.

Eine besonders hohe Stereoselektivität der Reduktion erhält man bei Verwendung von Aluminiumisopropylat in Isopropanol.

Die Reaktionstemperaturen können beim erfindungsgemäßen Verfahren in einem größeren Bereich variiert werden. Im allgemeinen arbeitet man zwischen etwa 80 und 120°C, vorzugsweise bei der Siedetemperatur des Lösungsmittels.

Bei der Durchführung der erfindungsgemäßen Umsetzung setzt man auf 1 Mol des Ketons der Formel (II) vorzugsweise C,35 bis 1,5 Mol an sekundärem Alkoholat ein. Zur Isolierung der Verbindung der Formel (I) wird das überschüssige Lösungsmittel durch Destillation im Vakuum entfernt und die entstandene Aluminium-Verbindung mit verdünnter Schwefelsäure bzw. Natriumhydrogensulfat-Lösung zersetzt. Die weitere Aufarbeitung erfolgt in üblicher Weise.

Nach einer bevorzugten Ausführungsform wird zweckmäßigerweise so verfahren, daß man das Keton der Formel (II) bereits in Gegenwart der entsprechenden Form A reduziert, wodurch die Stereoselektivität der Reduktion weiter zugunsten der gewünschten Form A verschoben wird. Dabei setzt man vorzugsweise auf 1 Mol des Ketons der Formel (II) 0 5 Mol der entsprechenden diastereomeren Form A und 0,5 Mol an sekundärem Alkoholat ein.

Zur Herstellung von Säureadditionssalzen der Verbindung der Formel (I) kommen alle physiologisch verträglichen Säuren infrage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure sowie Sulfonsäuren, wie z.B. p-Toluolsulfonsäure und 1,5-Naphthalindisulfonsäure.

Die Salze der Verbindung der Formel (I) können in einfacher Weise nach üblichen Salzbildungsmethoden, z.B. durch Lösen der Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.



BAD ORIGINAL

Die erfindungsgemäßen Verbindungen der Formel (I) und ihre Säureadditions-Salze weisen antimikrobielle, insbesondere antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkund der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiete in der Tiermedizin können beispielsweise aufgeführt werden:
Alle Dermatomykosen und Systemmykosen, insbesondere solche, die durch die oben genannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quartenäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirksoffen die üblichen Trägerstoffe enthalten, z.B. tierische und pfanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Aethylalkohol, Isopropylalkohol, Aethylcarbonat, Aethylacetat, Benzylalkohol, Benzylenzoat, Propylenglykol, 1,3-Butylenglykol, Dimethylformamid, Oele, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Aethylalkohol, Propylenglykol, Suspendiermittel, z.B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die gananten Formulierungsformen können auch Färbemittel, Konservierungsstoffe sowie geruchs- und geschmackverbessernde Zusätze, z.B. Pfefferminzöl und Eukalyptusöl und Süßmittel, z.B. Sacharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die

oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.



BAD ORIGINAL

Beispiel A

Antimykotische In-vitro-Wirksamkeit

Versuchsbeschreibung:

Die In-vitro-Wirksamkeit - gemessen anhand der minimalen Hemmkonzentrationen - wurde im Agarverdünnungstest ermittelt. Auf Serien von Kimmig - Agarplatten, die die entsprechenden Wirkstoffkonzentrationen im Agar gelöst enthielten, wurden mit Hilfe eines automatischen Beimpfungsgerätes Proben der Keimsuspensionen tropfenförmig aufgebracht. Das Inokulum betrug 2 - 5 x $10^3$ Pilzpartikel pro Impfpunkt. Im Falle der Hefen lag die Bebrütungsdauer bei 24 Stunden, im Falle der Dermatophyten bei 96 Stunden; Bebrütungstempertur 27 °C.

In dieser Versuchsanordnung zeigte das erfindungsgemäße Enantiomerengemisch der Formen A gegenüber zahlreichen Hefen, vor allem Candidarten, und Dermatophyten wesentlich bessere minimale Hemmkonzentrationen als das bekannte Diastereomerengemisch. Das Enantiomerengemisch der Form B erwies sich nur als schwach wirksam.

Beispiel B

Antimikrobielle In-vivo-Wirksamkeit (oral) bei Mäuse-Candidose

Mäuse vom Typ SPF-CF$_1$ werden intravenös mit 1-2 x $10^6$ logarithmisch wachsenden Candida-Zellen, suspendiert in physiologischer Kochsalzlösung, infiziert. Die Tiere werden mit Dosierungen von 12,5/25/50/100mg/kg Körpergewicht 2 x täglich über 5 Tage oral behandelt.

Ergebnis

Bei allen geprüften Dosierungen ist eine deutliche Abstufung der therapeutischen Wirksamkeit zu erkennen:

Form B (Enantiomerengemisch) < 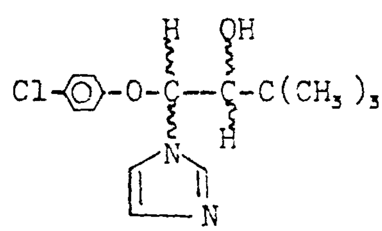 < Form A(1) (Enantiomerengemisch)

(Diastereomerengemisch/bekannt)



BAD ORIGINAL

Beispiel C

Antimikrobielle In-vivo-Wirksamkeit (lokal und oral) am Modell der experimentellen Meerschweinchen-Trichophytie

Versuchsbeschreibung:

Weiße Meerschweinchen der Rasse Pirbright-white werden auf dem geschorenen, nicht skarifizierten Rücken mit einer Mikro- und Makrokonidien-Suspension von Trichophyton mentagrophytes infiziert. Bei unbehandelten Tieren entwickelt sich innerhalb 12 Tagen p.i. das typische Bild einer Dermatophytose mit Rötung, Schuppung und Haarausfall bis zum totalen Integument-Defekt an der Infektionsstelle. Die infizierten Tiere werden

a) beginnend mit dem 3. Tag p.i. 1 x täglich mit 1%-iger Lösung der erfindungsgemäßen Präparate (in Dimethylsulfoxid : Glycerin = 1 : 4), lokal behandelt;

b) beginnend mit dem Tag der Infektion 1 x täglich mit 25 mg/kg Körpergewicht der erfindungsgemäßen Präparate (in wässriger Lösung) oral behandelt.

Das erfindungsgemäße Enantiomerengemisch der Form A erwies sich bei lokaler und oraler Therapie als gut wirksam; das Enantiomerengemisch zeigte keine Wirkung, während das bekannte Diatereomerengemisch eine Spur Wirkung hatte.

Herstellungsbeispiele

Beispiel 1

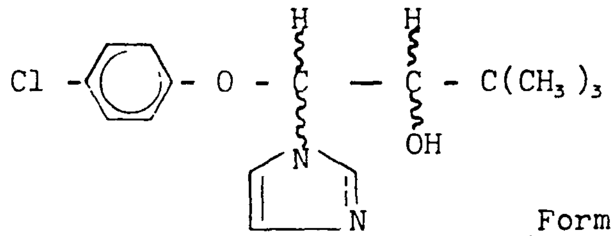

Form A
(Enantiomerengemisch)

10kg (34,16 Mol) 1-(4-Chlorphenoxy)-1-(1-imidazolyl)-3,3-dimethyl-2-butanon, 3,44 kg (16,74 Mol) Aluminiumisopropylat und 28 l Isopropanol werden in einem 40 l Glaskessel 16 Stunden bei einer Badtemperatur von 98 - 102°C erhitzt. Dabei werden 12 l Lösungsmittel abdestilliert. Das beim Abkühlen der Reaktionslösung ausfallende Produkt wird mit 20 l Isopropanol versetzt, auf 70°C aufgeheizt, heiß in einen 100 l Emaille-Kessel abgelassen und unter Rühren mit einer Lösung von 6,91 kg (57,6 Mol) Natriumhydrogensulfat in 32 l Wasser versetzt. Man läßt 8 Stunden rühren und saugt danach über Tonnutschen ab. Der feuchte Rückstand wird in 13 l Ethanol aufgeschlämmt und mit einer Lösung von 10,3 l 10%-iger Natronlauge und 25,7 l Wasser versetzt. Man läßt 4 Stunden rühren, saugt ab, versetzt den Rückstand mit 20 l Wasser, saugt erneut ab und trocknet. Man erhält 7,5 kg (66,7 % der Theorie) reine Form A (Gaschromatographisch bestimmt) von 1-(4-Chlorphenoxy)-1-(1-imidazolyl)-3,3-dimethyl-2-butanol vom Schmelzpunkt 158-159°C.

Beispiel 2

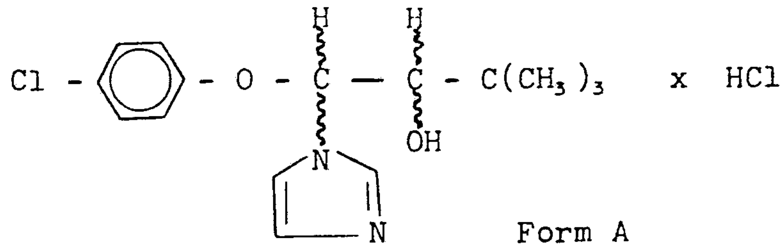

Form A
(Enantiomerengemisch)

Die gemäß Beispiel 1 erhaltenen 7,5 kg reine Form A von 1-(4-Chlorphenoxy)-1-(1-imidazolyl)-3,3-dimethyl-2-butanol werden in 37,5 l 2n-Salzsäure verrührt. Nach Zugabe von 50 l n-Butanol läßt man 2,5 Stunden rühren, trennt die organische Phase ab, wäscht zweimal mit 12,5 l 2n-Salzsäure nach und engt ein. Der Rückstand wird kalt mit 10 l Methylenchlorid aufgeschlämmt und abgesaugt. Der Rückstand wird mit 6 l Methylenchlorid nachgewaschen und bei 50°C im Umlüfter getrocknet. Man erhält 8 kg (95 % der Theorie) reine Form A (Gaschromatographisch bestimmt) von 1-(4-Chlorphenoxy)-1-(1-imidazolyl)-3,3-dimethyl-2-butanol-hydrochlorid vom Schmelzpunkt 236-37°C.

Patentansprüche:

1. Diastereomere Form A des 1-(4-Chlorphenoxy)-1-(1-imidazolyl-3,3-dimethyl-2-Butanols der Formel

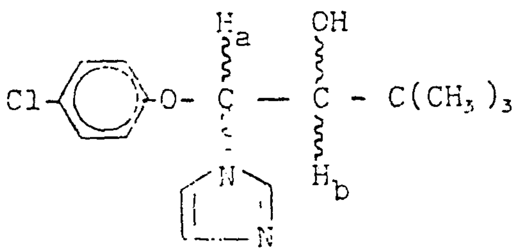
(I)

und deren physiologisch verträglichen Säureadditions-Salze.

2. Verfahren zur Herstellung der diastereomeren Form A des 1-(4-Chlorphenoxy)-1(1-imidazolyl)-3,3-dimethyl-2-butanols der Formel (I), dadurch gekennzeichnet, daß man 1-(4-Chlor-phenoxy)-1-(1-imidazolyl)-3,3-dimethyl-2-butanon der Formel

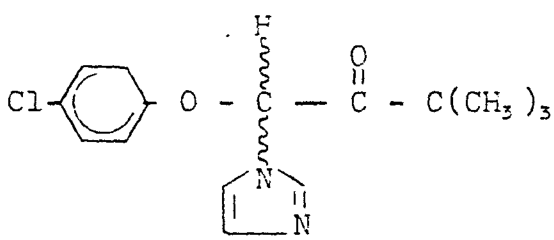
(II)

mit sekundären Alkoholaten in Gegenwart eines Verdünnungsmittels stereoselektiv reduziert und das Produkt gegebenenfalls durch Umsetzen mit Säuren in die Salze überführt.

3. Arzneimittel, gekennzeichnet durch einen Gehalt an mindestens einer diastereomeren Form A des 1-(4-Chlorphenoxy)-1-(1-imidazolyl)3,3-dimethyl-butanol und/oder deren physiologisch verträgliche. Säureadditionssalze gemäß Anspruch 1.

4. Verfahren zur Herstellung von antimykotischen Mitteln, dadurch gekennzeichnet, daß man diastereomere Form A des 1(4-Chlorphenoxy)-1-(1-imidazolyl)3,3-dimethyl-butanol und/oder deren physiologisch verträgliche Säureadditionssalze gemäß Anspruch 1 mit inerten, nichttoxischen, pharmazeutisch geeigneten Trägerstoffen vermischt.

5. Verfahren zur Behandlung von Mykosen, dadurch gekennzeichnet, daß man diastereomere Form A des 1-(4-Chlorphenoxy)-1-(1-imidazolyl)-3,3-dimethyl-butanols und/oder deren physiologisch verträgliche Säureadditionssalze gemäß Anspruch 1 Menschen oder Tieren appliziert, die an Mykosen erkrankt sind.

0011191

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 79 10 4254

| EINSCHLÄGIGE DOKUMENTE | | | KLASSIFIKATION DER ANMELDUNG (Int Cl ) |
|---|---|---|---|
| Kategorie | Kennzeichnung des Dokuments mit Angabe soweit erforderlich, der maßgeblichen Teile | betrifft Anspruch | |
| X | DE - A - 2 333 354 (BAYER)<br>* Seiten 1-8, 10-13, 20-22 *<br>-- | 1-4 | C 07 D 233/60<br>A 61 K 31/415 |
| D,X | DE - A - 2 333 355 (BAYER)<br>* Seiten 1-11,17-19 *<br>---- | 1-4 | |
| | | | RECHERCHIERTE SACHGEBIETE (Int Cl ) |
| | | | C 07 D 233/60<br>A 61 K 31/415 |

**UNVOLLSTÄNDIGE RECHERCHE**

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-4

Unvollständig recherchierte Patentansprüche:

Nicht recherchierte Patentansprüche: 5

Grund für die Beschränkung der Recherche: Verfahren zur chirurgischen oder therapeutischen Behandlung des menschlichen oder tierischen Körpers. (Siehe Art. 52(4) des Europäischen Patentübereinkommens).

KATEGORIE DER GENANNTEN DOKUMENTE

- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

| Recherchenort | Abschlußdatum der Recherche | Prüfer |
|---|---|---|
| Den Haag | 07-02-1980 | DE BUYSER |

EPA Form 1505.1 06.78